# EUROPEAN PATENT SPECIFICATION

(11) **EP 1 570 046 B1**
(45) Date of publication and mention of the grant of the patent: **05.03.2008**
(21) Application number: 03767637.6
(22) Date of filing: 25.11.2003
(51) Int. Cl.: C12N 1/04, C12N 11/02, C12N 11/14

(54) **METHOD AND DEVICE FOR DRYING OF MICRO-ORGANISMS**
VERFAHREN UND VORRICHTUNG ZUR TROCKNUNG VON MIKROORGANISMEN
PROCEDE ET DISPOSITIF DE SECHAGE DE MICRO-ORGANISMES

(30) Priority: 13.12.2002 EP 02027875
(43) Date of publication of application: 07.09.2005
(73) Proprietor: Merck Patent GmbH, 64293 Darmstadt (DE)
(72) Inventor: Rayner-Brandes, Michael Howard, 64665 Alsbach (DE); Watkins, Ian David, Cardiff CF15 8LT (GB); Jones, Julie Kay, Cardiff CF3 8AX (GB)
(86) International application number: PCT/EP2003/013205
(87) International publication number: WO 2004/055171

(56) References cited:
- EP-A- 0 298 605
- WO-A-01/05941
- WO-A-01/37656
- US-A- 4 886 664
- US-A- 4 888 171
- POPOVIC TANJA ET AL: "Evaluation of silica gel packages for transport Neisseria meningitidis" JOURNAL OF CLINICAL MICROBIOLOGY, vol. 36, no. 6, June 1998 (1998-06), pages 1765-1766, XP002271251 ISSN: 0095-1137 cited in the application
- MUGNIER J ET AL: "SURVIVAL OF BACTERIA AND FUNGI IN RELATION TO WATER ACTIVITY AND THE SOLVENT PROPERTIES OF WATER IN BIOPOLYMER GELS" APPLIED AND ENVIRONMENTAL MICROBIOLOGY, WASHINGTON,DC, US, vol. 50, no. 1, July 1985 (1985-07), pages 108-114, XP000978796 ISSN: 0099-2240
- LOUIS P ET AL: "SURVIVAL OF ESCHERICHIA COLI DURING DRYING AND STORAGE IN THE PRESENCE OF COMPATIBLE SOLUTES" APPLIED MICROBIOLOGY AND BIOTECHNOLOGY, SPRINGER VERLAG, BERLIN, DE, vol. 41, no. 6, 1 August 1994 (1994-08-01), pages 684-688, XP002059594 ISSN: 0175-7598

## Description

This invention is concerned with a simplified procedure for the substantially dry storage of micro-organisms at temperatures above freezing without the need to apply external drying/vacuum to the system. The method involves placing a small amount of the material containing the micro-organisms into a re-sealable strip device containing dry, water absorbent material of much greater mass in so doing rendering the micro-organisms substantially dry and stable in storage.

### Background Art

Very few micro-organisms are naturally able to withstand storage in the vegetative state without specific laboratory manipulation. One of the first laboratory methods developed for the maintenance of micro-organisms over long periods was continuously growing and subculturing the cells. However, this is a tedious, time-consuming process with a high chance of contamination by foreign organisms and may result in significant genetic change (e.g. loss of plasmids and/or other important genetic determinants). There are many other established methods, all based on reducing metabolic activity, for the long-term storage of micro-organisms in the vegetative state. They have in common either
(i) a very low storage temperature (often -60 ºC or lower) and/or
(ii) the removal of water from the micro-organisms in a sealable container by application of external physical processes (vacuum or heat) followed by sealing the container from later ingress of water vapour during storage.

An improvement over existing drying and storage methods would give significant benefits if it were to be performed without the complicated drying equipment needed for the removal of water from the micro-organisms by application of external physical processes, without pre-treatment of the cells and if it were to yield dried micro-organisms that could be stored at temperatures above freezing.

Some attempts have been made to dry cells at room temperature with the aid of desiccating chemicals such as P₂O₅, silica gel or CaCl₂. However, all of these methods, as disclosed in the literature, show major disadvantages.

B. Janning et al., Journal of Applied Bacteriology, 77 (1994), 319-324 investigated the drying of micro-organisms by directly contacting them with silica gel. Data reported by Janning et al showed a wide range of variation in the susceptibility of different bacterial strains to drying by this method, including data showing poor viability and recovery of Escherichia coli strains.

When directly contacting the cells with desiccating chemicals, as in this method, only some of the micro-organisms survive as the desiccating chemicals may be toxic to the micro-organisms. In addition, the hydration of desiccating chemicals such as silica gel gives heat which may also be lethal to micro-organisms. In the method of B. Janning et al, rehydration of the samples has to be performed very carefully at low temperatures for this reason.

J. Antheunisse et al., Antonie van Leeuwenhoek 47 (1981), 539-545, disclose the drying of micro-organisms in an exsiccator containing CaCl₂ and silica gel as desiccating chemicals. Direct contact between the micro-organisms and the desiccating chemicals is avoided by putting the micro-organisms in ampoules containing a folded strip of filter paper and provided with cotton stoppers. Drying could be achieved within one week. After this, the ampoules have to be removed from the dry environment of the exsiccator, exposing the cultures to the risk of absorption of atmospheric moisture in so doing, before sealing with paraffin wax for convenient long term storage. J. Antheunisse et al reported that immediately after drying the logarithmic exponent of number of viable cells had decreased by 2-3 units and that in some cases this initial decline in viability was even greater, reflecting that this drying method is not optimal for many bacterial strains.

T. Popovic et al., Journal of Clinical Microbiology, 36(6) (1998), 1765-1766, report the use of foil envelopes containing sterile silica gel. The micro-organisms are harvested with a polyester swab and the swab with the harvested micro-organisms is put within the foil envelope. T. Popovic et al reported survival of Neisseria meningitidis at room temperature, describing recovery by stating that all strains exhibited heavy growth for at least 4 days but also that moderate growth on average persisted until day 9, suggesting significant reduction in viability in a relatively short period at room temperature. Table 1 in T. Popovic et al further illustrates significant decrease in strain viability in storage at room temperature for up to 90 days.

Methods such as those disclosed by J. Antheunisse et al and by T. Popovic et al in which the micro-organisms are not directly contacted with the desiccating chemicals or any other water absorbent preparation result in elongated drying times, more complicated procedures and a more complicated design of the device. Published results for these methods indicate aspects in which the decline in viable count of the test micro-organisms is marked, indicating that these methods are not optimal or adequate for long term storage.

It has been found that in spite of the drawbacks of prior art a simple and convenient device and method for drying micro-organisms with the aid of desiccating chemicals can be realised if the micro-organisms are directly contacted with a dry water absorbent preparation further containing protective substances which stabilise the micro-organisms throughout drying and storage whilst, in addition, preferably, desiccating chemicals such as silica gel or molecular sieve materials are incorporated into the device without being in direct contact with the micro-organisms. In addition, the design of the device is chosen such that application of the micro-organisms, drying, storage and rehydration can be performed as rapidly and as conveniently and effectively as possible.

### Brief Description of the Invention

The present invention relates to a re-sealable strip device for drying and storage of viable micro-organisms without losing viability comprising
- a strip comprising a water absorbent preparation containing an amount of at least one water absorbent substance, which is able to absorb the water contained in the chosen volume of added micro-organism preparation such that the final amount of water after addition of the cells in the preparation(s) is less than 10 % w/w and at least one protective substance
- a re-sealable housing which when being sealed provides a closed, water-impermeable system around the strip.

In one embodiment of the invention the water absorbent preparation incorporates substances that have both protective and water absorbent properties.

In another embodiment of the invention the primary water absorbent preparation further comprises one or several absorbent bibulous foam or fibre-based pads or other matrices.

In one embodiment of the invention, the water absorbent preparation is in contact with or in proximity to a secondary water absorbent preparation.

In one embodiment of the invention, the secondary water absorbent preparation comprises a desiccating chemical such as silica gel or molecular sieve materials.

In a preferred embodiment of the invention the secondary water absorbent preparation has the format of one or several absorbent beads, tablets, coupons, labels, polymer films, plugs, foam or fibre-based pads enclosed within the strip device.

In a further preferred embodiment the re-sealable housing is a foil pouch.

In a further embodiment the re-sealable housing comprises one or more access windows over each of which there is a water impermeable cover that can be opened and re-sealed such that once re-sealed the device regains its water impermeable properties.

In a further embodiment the re-sealable housing has a label on to which details of the micro-organism preparation can be noted and/or recorded by means such as bar coding.

In a further embodiment the re-sealable housing has a secondary tear or peel seal which can be opened in order to aseptically remove the strip.

The present invention further relates to a process for the drying and storage of viable micro-organisms without losing viability, by
a) providing a re-sealable strip device according to the present invention
b) opening a re-sealable access (inoculation) window cover in the housing of the strip device to gain access to the strip
c) adding the micro-organism preparation to be dried through the said window on to the water absorbent preparation located on the strip
d) sealing the strip device by closing the re-sealable window cover on said housing
e) storing the strip device,
whereby the amount of the micro-organism preparation and the amount of the water absorbent preparation within the device are chosen such that after the addition of the micro-organism preparation the water absorbent preparation is able to absorb the water contained in the micro-organism preparation and remain substantially dry, in so doing rendering the micro-organisms substantially dry and bringing them into contact with the protective substances in order to be stable in storage.

In a preferred embodiment, after step e), the micro-organisms are recovered by
f) opening the strip device and removing the strip from the housing
g) rehydrating the micro-organisms.

In an embodiment of the method according to the present invention, in step e) the strip device is stored at a temperature between -70°C and 37°C.

In a preferred embodiment of the method according to the present invention, in step e) the strip device is stored at a temperature between 0°C and 30°C.

The present invention further relates to a kit for drying and storage of micro-organisms at least comprising a re-sealable strip device according to the method of the invention. Said kit may also include any required information on procedures and/or ancillary materials such as containers of relevant solutions such as buffers or solutions incorporating protective substances that may be needed to optimise said procedures to achieve maximum efficiency of the preservation procedures.

### Description of the Drawings

Figure 1 shows an example of a format for a re-sealable strip device according to the present invention.
Figure 2 shows a flow diagram representation of an example of the method according to the present invention used to preserve cells by storage in a re-sealable strip device.

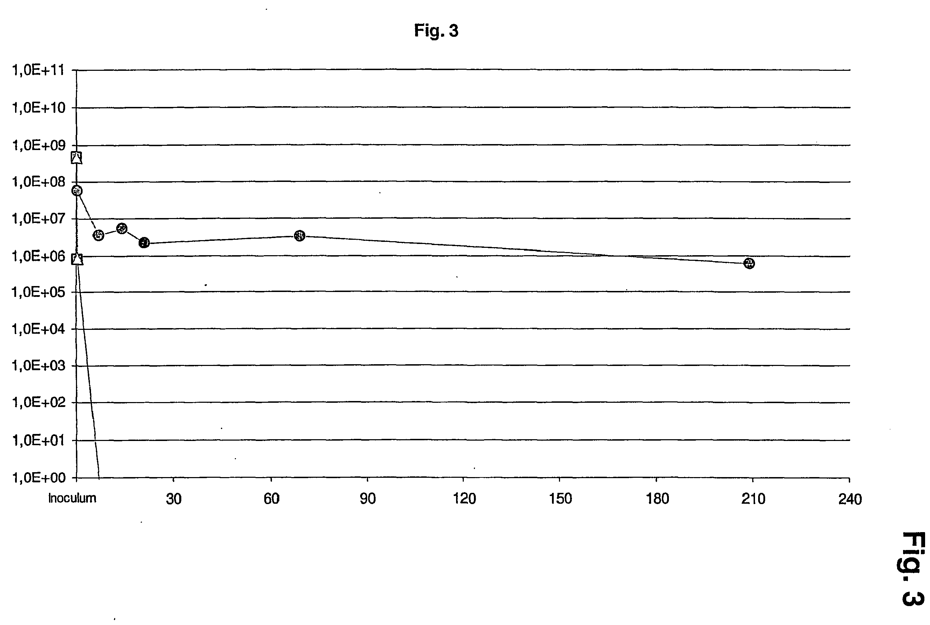

A detailed description of Figure 3 can be found in Example 1.

### Detailed Description of the Invention

This invention relates to a device and a method for drying micro-organisms for long-term storage. This is achieved by placing a preparation containing the micro-organisms (the micro-organism preparation) into a re-sealable strip device. The strip device comprises a water impermeable re-sealable housing and a strip within the housing. The strip comprises a solid support on to which is attached one or more water absorbent preparations. A water absorbent preparation comprises at least one or more water absorbent substances and one or more protective substances, whereby the volume of the micro-organism preparation and the amount and composition of the water absorbent preparation are chosen such that after the addition of the micro-organism preparation on to the water absorbent preparation and resealing of the test strip the said water absorbent preparation is able to absorb the water contained in the added micro-organism preparation and remain substantially dry, in so doing rendering the micro-organisms therein substantially dry and stable to storage.

The solid support is typically non-water absorbent material, often plastic such as polyester sheet. The water absorbent preparation is typically bonded to the solid support by adhesive. In another embodiment, the water absorbent preparation further comprises one or several absorbent bibulous foam or fibre-based pads or matrices being bonded to the solid support and incorporating one or more water absorbent substances and one or more protective substances.

According to the present invention, the expression "re-sealable device" means a device which is sealed to water ingress prior to use is such a way that within the water impermeable external housing of said device there are one or more holes (access windows) the water impermeable covers over which can be opened to allow access into the device then re-sealed to restore the barrier to water ingress.

The micro-organisms or cells that can be dried and stored according to the present invention include bacteria such as, but are not limited to, Enterobacteriaceae, such as Escherichia coli (including strains used for molecular biology procedures including transformation and including strains already transformed), Vibrionaceae such as Vibrio fischeri, organisms of industrial importance (Lactobacilli Spp.), organisms of medical relevance, Pseudomonadaceae, etc. Cells need not be in vegetative form but can also be in the spore state.

In another aspect of this invention, cells, for example E.coli NovaBlue (Novagen Inc., Madison, USA), can be dried using the method of the invention and then, after storage, rehydrated with a suitable competency inducing solution such that it is then possible to directly transform the cells with exogenous DNA, such as plasmid molecules, according to the method of WO 02/50252.

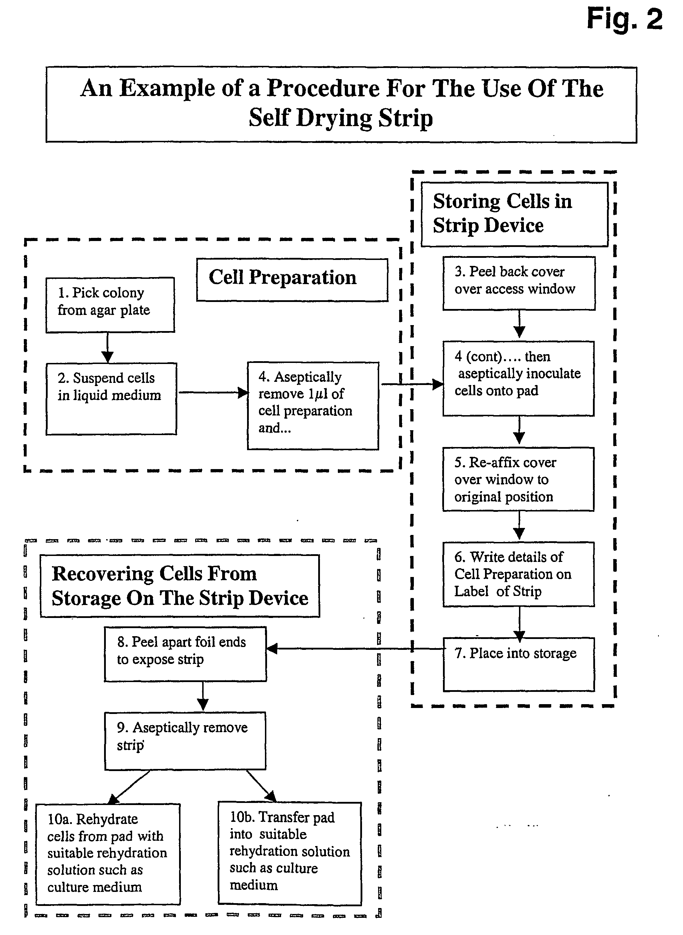

According to the invention, the sample of micro-organisms which is added to the device is called the micro-organism preparation, independently of how it is added (e.g. directly or after resuspension). Various sources of micro-organisms can be used, for example colonies, liquid-cultures, field samples, blood samples, etc. The micro-organism preparation can be obtained, for example, by sampling from a colony growing on a solid microbiological growth medium. This micro-organism preparation can be added direct onto the water absorbent preparation on the strip within the device or first suspended in a suitable solution before addition to the device, the latter shown in the flow diagram representation in Figure 2. Alternatively the micro-organism preparation can be obtained by sampling from a liquid culture or suspension of cells. In all cases above, as an example, a 1 µl loop can be used to transfer the micro-organism preparation into the device.

In all cases the micro-organism preparation can be pre-mixed with a solution containing protective substances prior to addition to the device. This may be achieved by dilution with such a solution or by centrifugation of the micro-organism preparation followed by re-suspension in such a solution. One can use the same protective substance(s) that is/are part of the water absorbent preparation or different protective substance(s).

As an example, a late logarithmic or stationary phase bacterial culture may contain approximately 10⁹ cells per millilitre, equivalent to 10⁶ cells per microlitre, so that very significant numbers of cells can easily be applied to the strip device in relatively low volumes of liquid.

The volume of the micro-organism preparation and the amount and type of the water absorbent preparation or preparations within the re-sealable strip device are chosen such that after the addition of the micro-organisms the water absorbent preparation is able to absorb the water contained in the added sample, in so doing rendering the micro-organisms substantially dry. Importantly this represents a closed system which is self-drying without external physical processes. Substantially dry means that the final amount of water after addition of the cells in the preparation(s) is preferably less that 10% w/w, more preferably less than 5% w/w and even more preferably less than 2% w/w water. The amount of water absorbent preparation needed depends upon the amount of micro-organism preparation to be dried and upon the water absorbing capacity of the material. A person skilled in the art is able to find out a suitable amount of water absorbent preparation so that it remains substantially dry after the addition of the micro-organism preparation and in doing so rendering the micro-organisms substantially dry.

These substantially dry conditions and careful choice of the water absorbent preparation and the protective substances therein enhance long-term stability of the micro-organisms, even at temperatures above freezing.

The water absorbent preparation that can be used to dry the cells according to the procedure of the present invention comprises at least one water absorbent substance and one protective substance. A protective substance is a substance which actively promotes maintenance of viability of the cells during and after drying.

The protective substances which are preferably incorporated into the water absorbent preparation and which are thus in contact with the micro-organisms are chosen to stabilise the cells during the drying process and minimise loss of viability. In particular, the protective substances assist in the preservation of the structural integrity of the cells in the dried state and also especially in the transition stages (drying and rehydration respectively) into and out of the dried state. Such protective substances may also act as water absorbent substances when incorporated into the water absorbent preparation.

Examples of protective substances include carbohydrates (either natural or synthetic), for example, sugars (e.g. sucrose, lactose, lactitol, trehalose, glucose, cellobiose, raffinose, cyclodextrin) and derivatives thereof and/or polymers (sugar and non-sugar based) and derivatives thereof and/or amino acids, peptides or proteins and derivatives thereof and/or protein-based preparations and fractions such as bovine serum albumin fraction V. This list is not intended to be comprehensive as such protective substances are well known to those skilled in the art.

The water absorbent substances can include any substance which is able to absorb water and whose properties (e.g. acidity, chemical reactivity) have no negative influence on the viability of the cells in the micro-organism preparation. Such material should not for example generate significant heat by exothermic reaction during the process of drying of the micro-organism preparation.

Specific examples for suitable water absorbent substances are dried carbohydrates or other organic or inorganic polymers like starch, dextrin, dextran, cellulose, protein, polypeptide, agar, agarose, polyacrilamide, sephadex, sepharose or mixtures thereof.

In one embodiment, the water absorbent substances additionally show the properties of protective substances, so that the water absorbent preparation may only comprise one substance which functions both as a water absorbent substance and as a protective substance. One example for such a substance would be a dried carbohydrate such as trehalose or sucrose.

In addition, the water absorbent preparation may be formulated to include other biologically compatible substances familiar to those skilled in the art such as biologically compatible buffer components, for example HEPES [N-[2-Hydroxyethyl]piprazine-N'-[2-ethanesulfonic acid], microbiological culture media or components of such media, activated charcoal, compatible solutes such as ectoines (reference: Louis P et al., Appl. Microbiol Biotechnol (1994) 41: 684-688), vitamin C or other biologically compatible reducing agents or antioxidants, and enzymes such as superoxide dismutase, peroxidase or chemicals which directly or indirectly neutralise free-radicals or neutralise chemicals which readily react to yield free-radicals.

Preferably, the water absorbent preparation consists of sugars such as trehalose and/or sucrose and one or more biologically compatible buffer components.

In another embodiment of the invention the water absorbent preparation has the format and thereby uses as a carrier of the protective substances one or several absorbent bibulous foam or fibre-based pads or matrices on the solid support incorporated within the strip device. Suitable pad materials include materials such as conventional cellulosic paper, nitrocellulose or derivatives thereof. Glass fibre materials, fibrous plastic materials such as Porex^{®} sheet materials (Porex Corporation), activated charcoal cloth and non-woven fabrics comprising such materials as viscose and polyester may also be used. All such materials above are well known to those skilled in the art.

In another preferred embodiment of the present invention, in addition to the water absorbent preparation (in the following, for ease of understanding called "primary water absorbent preparation"), the strip device further comprises a secondary water absorbent preparation. The secondary water absorbent preparation (either directly in contact with the primary water absorbent preparation or remote from it and in either case within the strip device) further enhances the efficiency of drying of the micro-organism preparation within the device. The primary water absorbent preparation is put directly in contact with the micro-organism preparation. If the amount of primary water absorbent preparation in relation to the amount of micro-organism preparation is too low to completely render the micro-organisms substantially dry or to ensure fast drying of the micro-organism preparation, the secondary water absorbent preparation is used to remove the excess water in the system. The amount of secondary water absorbent preparation is sufficient that the whole mixture becomes substantially dry. Typically, for a certain micro-organism preparation, the amount of primary and secondary water absorbent preparation taken together is equivalent to the amount of water absorbent preparation that would be needed if no differentiation was made and the whole water absorbent preparation was put directly in contact with the micro-organism preparation.

Primary and secondary water absorbent preparations can be made of the same or different water absorbent substances and can incorporate the same or different protective substances. They can have the same or different physical formats. Typically, the secondary water absorbent preparation need not incorporate any protective substances if it has only marginal contact with the micro-organism preparation.

Preferably, the secondary water absorbent preparation has the format of one or several beads, sachets, tablets, coupons, labels, polymer films, or pellets which may or may not be attached to the strip. It can be an advantage of such a combination of formats for the water absorbent preparations when, after storage and opening of the secondary seal of the water impermeable protective outer housing of the re-sealable strip device, the strip can then be easily separated from the secondary water absorbent preparation prior to rehydrating the micro-organism preparation from out of the primary water absorbent preparation. In this way the volume of solution needed to rehydrate the micro-organism preparation from out of the primary water absorbent preparation is lower than that required for rehydrating both the primary and secondary drying preparations. This has the further advantage that as less rehydration solution may be used, the resultant suspension of micro-organisms is more concentrated.

In another embodiment of the present invention water absorbent substances which do not need to be biologically compatible (desiccating chemicals) may be present within the device in secondary water absorbent preparations but physically separated from the micro-organisms. In this way desiccating chemicals which have excellent water absorbent properties can be deployed in the device in secondary water absorbent preparations without deleterious effects upon the micro-organism preparation caused, for example, by exothermic reaction upon hydration. Preferred examples of such desiccating chemicals which do not need to be biologically compatible are molecular sieves like zeolites, inorganic oxides like aluminium oxide or silica gel, montimorillonite clay or other materials known to those expert in the art.

In another embodiment of the present invention water absorbent substances or desiccating chemicals which do not need to be biologically compatible may be present within the device by incorporation of these substances into the material of the solid support of the strip.

The re-sealable housing of the strip device according to the present invention comprises a water impermeable protective outer packaging such as an aluminium foil pouch. Other water impermeable protective outer packaging materials are well known to those skilled in the art.

In a preferred embodiment, within the housing, there are one or more access windows over each of which there is a water impermeable cover that can be opened and re-sealed such that once this is done the device regains its water impermeable properties. Typically, the water impermeable cover would be secured to the device by suitable re-sealable adhesive at its periphery. The water impermeable cover could in this case take the form of a peelable tape.

Alternatively a hinge design can be conceived in which the cover is an integral part of the water impermeable protective outer packaging of the device and therefore secured by suitable re-sealable adhesive at only part of its periphery.

Alternatively a two-step mechanism can be conceived in which the primary cover over a given access window is peelable and removable and a secondary cover, such as a tape, is subsequently put into place over the access window so that the device regains its water impermeable properties.

In a further embodiment the water impermeable protective housing such as an aluminium foil pouch has a label on to which details of the micro-organism preparation can be noted and/or recorded by means such as bar coding.

In a further embodiment the housing has a secondary tear or peel seal which can be opened as required after storage in order to aseptically remove the strip holding the micro-organism preparation for reconstitution of the dried micro-organism preparation for use.

Typically, the contents of the re-sealable strip devices including the water absorbent preparation need to be sterile before adding the micro-organism preparation. The components of the devices may be sterilised separately prior to assembly or the devices may be sterilised subsequent to this but prior to adding the micro-organism preparation, for example using gamma irradiation or similar treatment. Thus there is no risk of contamination from micro-organisms which happen to have been introduced into the system during manufacture of the devices.

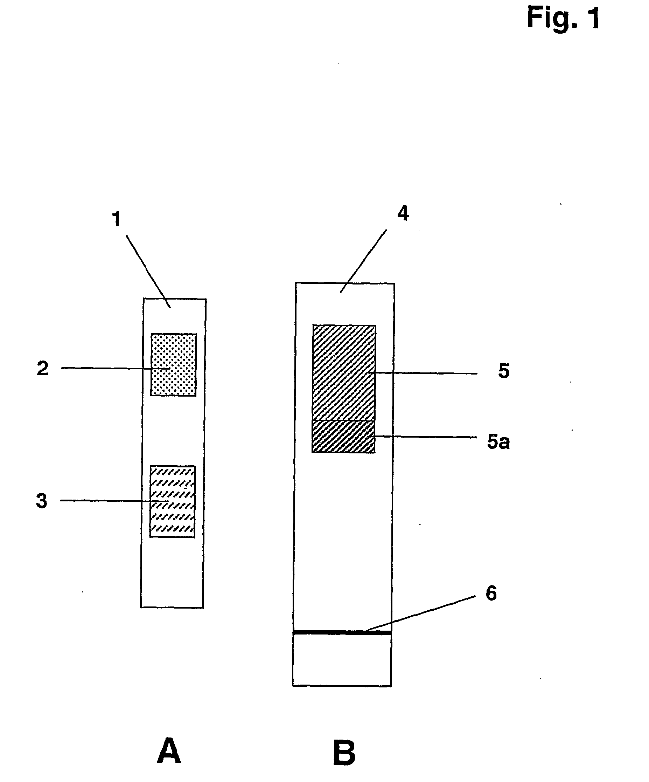

Figure 1 shows an example of a format for a re-sealable strip device according to the present invention. Part A shows the strip, part B the housing. The strip comprises a plastic strip (1) on to which is attached the water absorbent preparation (2) comprising a pad with at least one water absorbent substance and at least one protective substance. A secondary water absorbent preparation (3) comprising a pad with at least one water absorbent substance is located further down the strip.

The housing comprises a foil pouch (4) with one re-sealable access window (5). When the access window is peeled open at the peelable seal (5a) one gains access to the water absorbent preparation on the strip to be able to inoculate the micro-organism preparation on to the water absorbent preparation. For aseptically removing the strip from the housing, the housing can be opened by tearing the tear line (6).

The invention further relates to a process for the drying and storage of viable micro-organisms by
a) providing a re-sealable strip device according to the present invention
b) opening a re-sealable access (inoculation) window cover in the housing of the strip device to gain access to the strip
c) adding the micro-organism preparation to be dried through the said window on to the water absorbent preparation located on the strip
d) sealing the strip device by closing the re-sealable window cover on said housing
e) storing the strip device,
whereby the amount of the micro-organism preparation and the amount of the water absorbent preparation within the device are chosen such that after the addition of the micro-organism preparation the water absorbent preparation is able to absorb the water contained in the micro-organism preparation and remain substantially dry, in so doing rendering the micro-organisms substantially dry and bringing them into contact with the protective substances in order to be stable in storage.

The sealed strip devices with micro-organisms dried according to the present invention can be stably stored at temperatures between -70°C and +37°C, preferably between 0°C and +30°C. Cells which are "stably stored" are able to withstand storage for extended periods of time at a suitable temperature, without appreciably losing their viability. The storage period of time may range from about 0 days to about 2000 days, typically from about 20 days or less to about 500 days, although even longer storage times may be used at temperatures of less than about -20 °C.

After storage, the micro-organisms are recovered by
f) opening the strip device and removing the strip from the housing
g) rehydrating the micro-organisms.

Rehydration is typically performed either by the addition of a suitable reconstitution solution to the water absorbent preparation holding the preserved micro-organism preparation or by insertion of the test device into a suitable solution such as a microbiological culture medium or buffer solution.

One example for the procedure of the method according to the present invention is shown in Figure 2. In step 1, cells are removed from a colony of micro-organisms on an agar plate. In step 2, the cells are suspended in a small amount of sterile liquid medium which may contain protective substances. In step 3, the re-sealable window cover on the strip device is opened to gain access to the pad of the re-sealable strip device. In step 4, a small volume (1µl in this example) of the micro-organism preparation to be dried is taken and inoculated through the window on the device on to the water absorbent preparation in the pad on the strip device. In step 5, the strip device is straight away re-sealed by closing the re-sealable window cover on the test strip device. In step 6, the details of the micro-organism preparation are entered on to the label on the external packaging of the strip device. In step 7, the test strip is put into storage (for example in a fridge at 4°C) prior to use. In step 8, the strip device is opened in order to gain access to the preserved bacteria by opening a second seal, in this example a peel seal. In step 9, the strip device is then removed from its packaging. In step 10, the cells are rehydrated by the addition of a suitable rehydration solution either by direct addition to the pad (step 10a) or by insertion of the pad of the test device into a suitable rehydration solution (step 10b). In either step 10a or step 10b a microbiological culture medium could serve as a suitable rehydration solution.

In another example for the procedure of the method, step 2 may be omitted so that in the next step, the re-sealable window cover on the strip device is opened to gain access to the pad of the re-sealable strip device and then the cells removed from a colony of micro-organisms on an agar plate inoculated direct through the window on the device on to the water absorbent preparation in the pad on the strip device without prior suspension in liquid.

In another example for the procedure of the method, steps 1 and 2 may be omitted so that in the next step, a small volume (1µl in this example) of a micro-organism preparation that is a pre-existing liquid suspension such as a broth culture is taken and inoculated through the window on the device on to the water absorbent preparation in the pad on the strip device. Such a suspension may contain added protective substances. Alternatively such a suspension may be constituted by prior centrifugation of a broth culture or other suspension of micro-organisms and resuspension of the micro-organisms in a solution of choice which may include protective substances. These additional preparatory steps of centrifugation and resuspension may be used to adjust the concentration of micro-organisms in the suspension inoculated through the window on the device on to the strip device, typically to concentrate the micro-organisms.

In another aspect of the invention, a kit is provided for simple and instant drying of micro-organisms according to the method of the present invention. The kit comprises at least one re-sealable strip device containing at least one water absorbent preparation incorporating one or more protective substances according to the present invention. Preferably, the water absorbent preparation is configured in the format of one or several absorbent bibulous foam or fibre-based pads or other matrices deployed on the strip enclosed within the strip device so that the user simply has to open the cover of the access window of the device, add the micro-organism preparation containing micro-organisms to the pad and re-seal the access window of the device for storage. Said kit may also include any required information on procedures and/or ancillary materials such as containers of relevant solutions such as buffers or solutions incorporating protective substances that may be needed to optimise said procedures to achieve maximum efficiency of the preservation procedures.

The device and method according to the present invention eliminate the need for applying external drying equipment after adding the micro-organisms. Drying of the preparation(s) in the system is performed before the organisms are added, thus, the system is pre-prepared, sterilised (if required), and stored (without requirement for temperature controlled storage conditions) before use. The system thus has very few restrictions on when and where the micro-organism preparation sample is added, i.e. it is very flexible. Generally, storage at 4 ºC or below is recommended for long term storage after addition of the micro-organism preparation although this may depend on the organism.

For the user, storage within the laboratory at temperatures above 0 °C is both more convenient and user-friendly and also more economic. Furthermore, the shipment of cells preserved in the way according to the present invention can be achieved without freezing and is therefore also more convenient.

Compared to conventional storage methods, the main advantage of the self-drying methodology according to the present invention is the simplicity and convenience of the procedures involved in that typically the user only has to add a small amount of micro-organism suspension (or semi-solid material e.g. a colony) and then place in a refrigerator for storage. For example, it is not necessary to prepare sterile solutions with glycerol, a prerequisite for typical storage in freezers or to store the cells over liquid nitrogen, thus, the process is simpler, safer and faster. In addition, the device is a closed system so there is no significant time period in which the device is open to atmosphere during the process of drying of the micro-organism preparation. This is in marked contrast to, for example, many freeze drying processes in which aerosols are likely during the drying process, thus the present invention makes the process of handling and drying pathogenic organisms much safer.

Without further elaboration, it is believed that one skilled in the art can, using the preceding description, utilise the present invention to its fullest extent. The preferred specific embodiments and examples are, therefore, to be construed as merely illustrative, and not limiting to the remainder of the disclosure in any way whatsoever.

### Examples

### Experiment 1 To show cell survival on test strip with protectant on the pad

### Preparation of the strip devices

Sealed test strips from HY-RiSE^{™} Colour Hygiene Test Strips (Merck KGaA Art No. 1.31200.0001) were adapted in order to produce the self drying storage strips. Using a scalpel, an access window was cut into the foil pouch enclosing the test strip allowing access to the test strip pad. This was carried out by making a cut on 3 sides of an area 5 mm x 5 mm. The foil pouch was cut open at the end furthest end from the pad and the strip removed. Approximately 1cm of the plastic strip was trimmed from the end distal from the pad. An 86*µ*l aliquot of protectant (50% (w/w) Sucrose (BDHArt.no. 102745C) in Nutrient Broth NB (Nutrient Broth No.2, Merck KGaA, Art. no 10136) filter sterilised), was dispensed onto the pad and the strip placed beneath a hot air fan (Sunhouse 2000 made by T.I Sunhouse Ltd) for 30 minutes. The hot air fan was suspended above the bench in such a manner that the grill from which the hot air was blown was pointing downwards. The distance from the bench top to the grill was 350mm. The fan heater was switched onto maximum heat and resulted in an area of approximately 190mm x 270mm wherein the temperature achieved was 75-95°C. The strips to be dried were placed within this area upon a plastic grid (Denleystor, cryostorage ABS plastic gridded rack, 150mm x 150mm, with grids placed 5mm apart) raising the strips above the bench by 20mm. The strips were prepared and dried in batches of 36.

Self adhesive desiccant (CSP Technologies, ACTIV-STRIP, M-0002-58,) was removed from the protective foil pouch, cut into 3 pieces of 44.0mm x ~2.6mm x 0.60mm and placed immediately into a tin containing sachets of dry silica desiccant beads (Sigma, Silica beads for desiccation, Type II, size 1/8 "beads). After the pads on the strips were dried the desiccant pieces were removed from the tin and attached to the strips (see Figure 1 for position). The strips were then returned to the foil pouches and the pouches placed in a second tin containing dry silica desiccant beads for interim storage. The pouches were removed from this tin and their cut ends were foil sealed using a Multivac A300 Packager. The packager was programmed to draw a vacuum of approximately 600mbar with heat/pressure seal time of 3.5 seconds, sealing pressure 1 atmosphere. The pouches were then placed into a third tin containing dry silica desiccant. The pouches were removed from the tin and a strip of self adhesive plastic film (Tenza Clearview Film Art No. 329111) placed over the access window. The strips were held at room temperature for at least 16 hours prior to inoculation.

### Preparation of the inoculum of concentrated cells

A primary culture of *E.coli* JM109, a K12 derivative, (Deutsche Sammlung von Mikroorganismen und Zellkulturen GmbH {German Collection of Microorganisms and Cell Cultures} DSM 3423) was prepared as follows: JM109 cells were streaked on an LB (10g/l Tryptone (peptone from casein - Merck KGaA., Art. No. 1.07213), 5g/l Yeast Extract (Merck KGaA., Art No. 1.03753), 10g/l NaCl (BDH, Art No 10241), 15g/l Agar (Merck KGaA., Art No.536025J), pH adjusted to 7.5 with NaOH), agar plate, and the plate was incubated for 72 hours at 20 °C.

From this a single colony was inoculated into a 30ml Universal vial (Sterilin, Art. No.128A) containing 3ml SOB medium (20g/l Tryptone (peptone from casein - Merck KGaA., Art. No. 1.07213), 5g/l Yeast Extract (Merck KGaA., Art No. 1.03753), 0.5g/l NaCl (BDH, Art No 10241), 0.186g/l KCI (BDH, Art No.101984L)) supplemented with 30*µ*l 2M Mg²⁺ solution (1M MgSO_{4.}7H₂O (Fluka, Art No. 63138), 1M MgCl_{2.} 6H₂O (Fluka, Art No. 63068)), and incubated at 37°C. The resulting culture (primary culture) was grown to an OD₆₅₀ₙₘ of 0.40 (Pharmacia LKB, Novaspec II Visible Spectrophotometer), transferred to +4°C, and stored overnight.

To create a seed stock a 1ml aliquot of the primary culture was used to inoculate 50ml SOB medium supplemented with 500*µ*l 2M Mg²⁺ (1M MgSO_{4.}7H₂O (Fluka, Art No. 63138), 1M MgCl_{2.} 6H₂O (Fluka, Art No. 63068) solution in a 250ml conical flask (Pyrex, Art. No. 1140/14) and incubated at 37°C, with shaking at 250rpm. The OD₆₅₀ₙₘ was followed until the culture was at 0.65. An aliquot of 35ml SOB medium (20g/l Tryptone (peptone from casein - Merck KGaA., Art. No. 1.07213), 5g/l Yeast Extract (Merck KGaA., Art No. 1.03753), 0.5g/l NaCl (BDH, Art No 10241), 0.186g/l KCI (BDH, Art No.101984L)) supplemented with 350*µ*l 2M Mg²+ (1M MgSO_{4.}7H₂O (Fluka, Art No. 63138), 1M MgCl_{2.} 6H₂O (Fluka, Art No. 63068), was added to the flask containing the culture and swirled gently to mix. The culture was dispensed in 850*µ*l aliquots into pre-chilled cryovial vials (NUNC, Art.No. 3.68632). An aliquot of 150*µ*l glycerol (Fluka, Art No. 49767), was added to each vial, the content mixed by gently inverting, and placed immediately on dry ice. When the contents of each seed stock vial were frozen they were transferred to - 70°C storage.

To prepare the inoculum, a vial of seed stock JM109 was removed from - 70°C storage. Using a 10 *µ*l loop, a sample was removed by scraping the surface of the content of the vial. The sample was spread on a Luria Broth Agar plate (10g/l Tryptone (Difco, Art. No. 211705), 5g/l Yeast Extract (Difco, Art No. 212750), 10g/l NaCl (Sigma, Art No. S7653), 15g/l Agar (Merck, KGaA, 536025J), pH adjusted to 7.5 with NaOH), using conventional methods to ensure growth of individual separate single colonies of bacteria. The plate was incubated for 16 hours at 37°C and transferred to +4°C storage.

The above plate was removed from +4°C storage and 3 colonies transferred to a 1L (Pyrex, Art No. 1130/26) conical flask containing 100ml LB broth (10g/l Tryptone (Difco, Art No. 212750), 5g/l Yeast extract (Difco, Art No. 212750), 10g/l NaCl (Sigma, Art No. S7653), - pH adjusted to 7.5 with NaOH). Two flasks were prepared in this manner. The flasks were incubated at 37°C with shaking at 180rpm. After approximately 16 hours the cultures reached an OD₆₀₀ₙₘ (Pharmacia LKB, Novaspec II Visible Spectrophotometer) of 1.49 and 1.45. The contents of the flasks were pooled and immediately placed on ice for 15 minutes.

Aliquots of 45ml of the chilled culture were transferred to 2 pre-chilled centrifuge tubes (Falcon, Art. no. 352070). The cells were collected by centrifugation in an 5804R Eppendorf centrifuge using rotor type F34-6-38 and inserts (Eppendorf, Art No 5804 774000), at 6000rpm, 4°C for 5 minutes. An aliquot of approximately 5ml of the supernatant (ie spent media) was decanted into a Universal vial and the remainder discarded, taking care not to disturb the pellet. The Universal vial containing the supernatant was placed on ice. The pellets were each resuspended in 0.45ml of the supernatant by gentle aspiration using a 1 ml sterile plastic pasteur pipette. The resuspended pellets were pooled, and returned to the ice bath. This concentrated cell suspension was used as the inoculum.

The viability of the cells in the inoculum was determined at this point. A standard dilution series was prepared in NB and plated in duplicate onto Nutrient Agar, NA (Merck KGaA, Art. no. 1.05450). The plates were incubated at 37°C for at least 18hours, or at 20°C for at least 72hours and the colonies counted.

### Inoculation of the strips

The cells used for the inoculum were maintained in the ice bath for the duration of this procedure. The adhesive film sealing the access window was peeled back allowing access to the pad. An aliquot of 1µl of the cell suspension was dispensed onto the pad using standard micropipettes (BRAND TRANSFERPETTE 0.1-1µl, PLASTIBRAND Tips, 0.1-20*µ*). The adhesive film was replaced immediately after inoculation and the access window thus re-sealed. This procedure was repeated for each of the strips to be inoculated. The inoculated strips remained at room temperature (ie 20°C) for 1 hour. As controls, six strips were tested to determine the average initial viable cell number on the strip according to the procedure below. The remaining strips were then transferred to elevated (30°C) temperature storage for defined periods. As required, after set time periods at the storage temperature, the strips were removed from this incubation, 6 in total for each time point, equilibrated to room temperature, then tested to determine the remaining viable cell number on the strip according to the procedure below.

### To determine the viable cell number on the strip.

The foil pouch was opened by tearing the foil at the opposite end to the inoculated pad. The strip was aseptically removed and transferred to a Bijou (Sterilin Art. no. 129A) containing 2ml NB in order to immerse the pad. The Bijou and contents were gently agitated and a 5 minute period allowed for the cells/protectant to re-hydrate from the pad. A standard dilution series was prepared in NB and plated in duplicate onto Nutrient Agar, NA (Merck KGaA, Art. no. 1.05450). The plates were incubated at 37°C for at least 18 hours, or at 20°C for at least 72hours and the colonies counted.

### Control A - Strips prepared without protectant on the pad and no protectant in the inoculum

Strip devices were prepared and tested as described in the above method, except for the omission of the dispensing and drying of protectant on the pad. The strip device was therefore also not placed for 30 minutes beneath the hot air fan.

### Control B - Strips prepared with No protectant on the pad. Protectant present in the inoculum.

Strip devices were prepared and tested as described in the above method, except for the omission of the dispensing and drying of protectant on the pad and except for the following modification to the preparation of the inoculum.

The inoculum was prepared as described above except that each pellet was resuspended in 0.45ml protectant solution (25% (w/w) Sucrose (BDH Art.no. 102745C) in Nutrient Broth (Nutrient Broth No.2 Merck KGaA, Art. no. 110136), filter sterilised)

### Results:

The surprising result can be seen from Table A below that good stability (survival for greater than 209 days (in elevated (30°C) temperature storage) was only achieved with the incorporation of protectant on the pad of the device. The bacteria inoculated into the device did not survive for greater than seven days in the absence of dried protectant on the pad.

**Table A**

| | | | **Control A** | **Control B** |
|---|---|---|---|---|
| | **Time (days)** | **Protectant on pad / NO protectant in the inoculum** (cfu/strip) | NO protectant on the pad / NO protectant in the inoculum (cfu/strip) | NO protectant on the pad / Protectant in the inoculum (cfu/strip) |
| **Inoculum added to strip** | 0 | **4.60E+08** | **4.60E+08** | **4.45E+08** |
| | | | | |
| **Cells recovered from storage strip** | 0 (1 hr after inoculation) | 5.5E+07 | 7.7E+05 | 8.4E+05 |
| | 7 | 3.4E+06 | No colonies observed | No colonies observed |
| | 14 | 5.1E+06 | No colonies observed. | No colonies observed. |
| | 21 | 2.2E+06 | No further tests carried out | No further tests carried out |
| | 69 | 3.1E+06 | | |
| | 209 | 6.1E+05 | | |

A graphical view of these results can be found in Figure 3. On the X-axis the time is given in days. On the Y-axis, the cfu per strip is shown. The circles show the data obtained with a test strip according to the present invention (Protectant on pad / NO protectant in the inoculum), the quadrats and triangles show the data obtained with Control A and Control B respectively.

### Experiment 2 To show cell survival with Campylobacter jejuni ss. jejuni ATCC -33560 on the test strip, using 3 inoculum types; Colony, Culture and Concentrated Cells.

### Preparation of the strip devices

As described for Experiment 1

### Preparation of the inocula

Approximately 6ml Bolton Broth (Oxoid, Art. No. CM983, autoclaved at 121°C for 15 minutes) plus 5% Laked Horse Blood (SR0048C, added to the broth once cooled to below 50°C) in a 7ml Bijou was inoculated with *Campylobacter jejuni ss. jejuni,* ATCC33560, cells and incubated at 37°C for 72 hours. This culture was maintained as a serial culture by transferring 1 ml culture into 5ml Bolton Broth plus 5% Laked Horse Blood, contained in Bijou, and incubated at 37°C for 48-72 hours.

An aliquot of 1 ml of the above culture was used to inoculate 5ml Bolton Broth (without blood) in a Bijou, and incubated at 37°C for 48-72 hours. This culture was maintained as a serial culture by transferring 1 ml culture into 5ml Bolton Broth (without blood), contained in a Bijou, and incubated at 37°C for 48-72 hours.

From this culture, cells were streaked on to a Columbia Blood Agar plate, (39g/l Columbia Agar Base, Oxoid CM331, sterilised at 121°C for 15 minutes and 5% Laked Horse Blood, Oxoid SR0048C added when cooled to below 50°C) and incubated at 37°C for 48 hours in a CO₂ enriched atmosphere. This plate was used to inoculate the strips with colonies (the colony inoculum).

Cultures were set up from the serial culture by inoculating 6 x 5ml Bolton Broth (without blood) contained in Bijou, each with 1 ml culture. The cultures were grown at 37°C for 48 hours.

The contents of the Bijou were pooled in a 30ml Universal vial (Sterilin, Art. No.128A) and immediately placed on ice for 15 minutes. The OD₆₀₀ₙₘ (Pharmacia LKB, Novaspec II Visible Spectrophotometer) of the pooled culture was measured to be 0.21. An aliquot of approximately 3ml of this culture was maintained on ice and used to inoculate the strips (the Culture inoculum).

Aliquots of 2 x 13ml of the chilled culture were transferred into 2 pre-chilled centrifuge tubes (Falcon, Art. no. 352096). The cells were collected by centrifugation in an 5804R Eppendorf centrifuge using rotor type F34-6-38 and inserts (Eppendorf, Art No 5804 774000), at 6000rpm, 4°C for 5 minutes. An aliquot of approximately 5ml of the supernatant, (ie spent media) was decanted into a Universal vial and the remainder discarded, taking care not to disturb the pellet. The Universal containing the supernatant was placed on ice. The pellets were resuspended, each with 0.13ml of the chilled supernatent by gentle aspiration using a 1ml sterile plastic pasteur pipette. The concentrated cell suspensions were pooled and used to inoculate the strips (the Concentrated cell inoculum).

The viability of the cells in the Culture inoculum and the Concentrated cell inoculum were determined at this point. Standard dilution series were prepared in Bolton Broth (without blood) and plated in duplicate onto Columbia Blood Agar. The plates were incubated at 37°C for at least 48 hours in a CO₂ enriched environment, and the colonies counted.

### Inoculation of the strips

### Inoculation using colonies

The plate from which single colonies were to be used was removed from the incubator and placed onto the workbench at 20°C. The seal on a strip was peeled back allowing access to the pad via the access window. Each colony was removed from the agar plate using a *µ*l loop (Nunc Brand, 1*µ*l Clear, Art No. 254410) and "rubbed" onto the pad/protectant. The seal was replaced immediately. This procedure was repeated for each of the strips to be inoculated.

The cell viability of a typical colony from the plate was determined at this point. The colony was resuspended in 1 ml of Bolton Broth (without blood), a standard dilution series was prepared in the same broth, plated in duplicate onto Columbia Blood Agar and incubated at the required temperature and duration. The colonies were counted to establish the cfu per colony.

### Inoculation using the culture and the concentrated cell suspension

As described for Experiment 1.

This procedure was carried out for each of the inoculum types.

### To determine the viable cell number on the strip.

As described in Experiment 1 with the following exceptions;

Bolton Broth (without blood) was used as the diluent, and Columbia Blood Agar as the growth medium. The plates were incubated at 37°C for at least 48-72 hours in a CO₂ enriched atmosphere.

### Control C - Strips prepared as described above without inoculation

Strip devices were prepared and tested as described in the above method, except for the omission of any kind of inoculum. The strips were tested, 36 in total, as described above for the determination of cell viability after storage.

### Results:

Table B below shows good stability (survival for greater than 56 days) in elevated (30°C) temperature storage using the method of the invention.

**Table B**

| | **Time (days)** | **Strips inoculated with single colonies** (cfu/strip) | **Strips inoculated with stationery culture** (cfu/strip) | **Strips inoculated with concentrated cells** (cfu/strip) | **Control C No inoculation** (cfu/strip) |
|---|---|---|---|---|---|
| **Inoculum added to strip** | 0 | **1.5E+08** | **9.0E+05** | **3.9E+06** | No colonies observed. |
| | | | | | |
| **Cells recovered from storage strip** | 0 (1 hr after inoculation) | 1.1 E+06 | 6.1 E+03 | 1.3E+05 | No further tests carried out |
| | 7 | 6.5E+04 | 2.7E+03 | 1.4E+05 | |
| | 14 | 7.8E+04 | 3.1 E+03 | 6.6E+04 | |
| | 21 | 6.5E+04 | 2.4E+03 | 1.1E+05 | |
| | 56 | 4.3E+03 | 1.7E+03 | 5.9E+04 | |

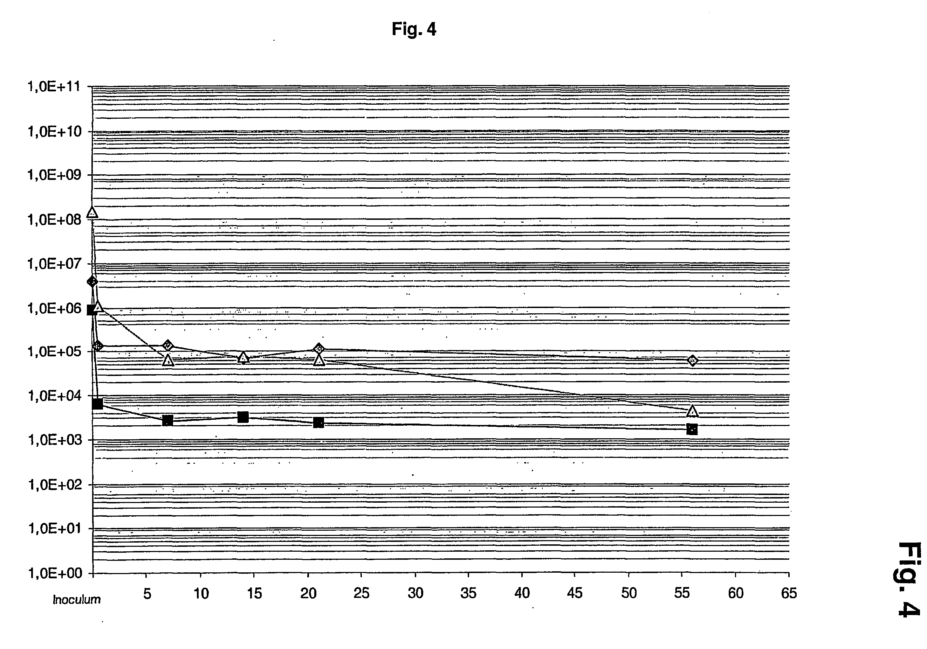

A graphical view of these results can be found in Figure 4. On the X-axis the time is given in days. On the Y-axis, the cfu per strip is shown. The triangles quadrats, and diamonds show the data obtained with the Singles colony, Culture and Concentrated cell inoculums respectively.

### Experiment 3 To show cell survival with Candida albicans ATCC - 10231 on the test strip, using 3 inoculum types; Colony, Culture and Concentrated cells.

### Preparation of the strip devices

As detailed in Experiment 1 with the following exception;

The self adhesive desiccant (CSP Technologies, ACTIV-STRIP, M-0002-58). was removed from the protective foil pouch, and cut into pieces of 44.0mm x ~12.6mm x 0.30mm.

### Preparation of the inocula

*Candida albicans* ATCC-10231 was obtained as TWISTER 0006 from which a seed stock was prepared. The TWISTER was rehydrated using the rehydration solution provided, streaked onto the Sabouraud 4% Maltose agar (65g/l Merck Art. no. 105439) and incubated at 25°C for 72 hours.

A well isolated colony was selected from the agar plate, transferred to a vial containing cryobeads (Microbank, PROLAB Diagnostics, Art. No. PL160) and emulsified in the protective medium. The vial was inverted several times and as much of the protectant medium as possible was removed by aspiration. The vial was transferred to -70°C immediately after inoculation.

The organism was streaked from this cryobead stock onto a Sabouraud 4% Maltose Agar plate using conventional methods to ensure growth of individual separate single colonies of bacteria, and incubated at 25°C for 72 hours. This agar plate was used to provide single colonies for the inoculation of the strips (the Colony inoculum), and to inoculate broth to be used for the Culture and Concentrated cell inoculums.

A single colony from the plate prepared above was used to inoculate 50ml Maltose Broth (10g/l Peptone, Merck Art. No. 107213, 40g/l Maltose, Merck Art. No. 291314H) in a 100ml Sterilin pot (Sterilin Art. No. 185AM), incubated at 25°C for 72 hours. The optical density, OD₆₀₀ₙₘ was measured (Pharmacia LKB, Novaspec II Visible Spectrophotometer) to be 0.86. The culture was placed on ice for 15 minutes. An aliquot of approximately 4ml of this culture was maintained on ice and used to inoculate the strips (the Culture inoculum).

An aliquot of 45ml of the chilled culture was transferred to a pre-chilled centrifuge tube (Falcon, Art. no. 352070). The cells were collected by centrifugation in an 5804R Eppendorf centrifuge using rotor type F34-6-38 and inserts (Eppendorf, Art No 5804 774000), at 6000rpm, 4°C for 5 minutes. An aliquot of approximately 5ml of the supernatant (ie spent media) was decanted into a Universal vial and the remainder discarded, taking care not to disturb the pellet. The Universal containing the supernatant was placed on ice. The pellet was resuspended in 0.45ml of the supernatant by gentle aspiration using a 1ml sterile plastic pasteur pipette. The resuspended pellet was pooled, and returned to the ice bath. This concentrated cell suspension was used to inoculate the strips (the Concentrated cell inoculum).

The viability of the cells in the Culture inoculum and the Concentrated cell inoculum were determined at this point. Standard dilution series were prepared in Maltose Broth and plated in duplicate onto Sabouraud 4% Maltose agar. The plates were incubated at 25°C for at least 72 hours, and the colonies counted.

### Inoculation of the strips

### Inoculation using colonies

As described in Experiment 2 with the following exceptions:
Maltose broth was used as the diluent, and Sabouraud 4% Maltose Agar as the growth medium. The plates were incubated at 25°C for at least 72 hours.

### Inoculation using the culture and the concentrated cell suspension

As described for Experiment 1.

This procedure was carried out for each of the inoculum types.

### To determine the viable cell number on the strip.

As described in Experiment 1 with the following exceptions;

Maltose broth was used as the diluent, and Sabouraud 4% Maltose Agar as the growth medium. The plates were incubated at 25°C for at least 72 hours.

### Control D - Strips prepared as described above without inoculation

As described for Experiment 2.

### Results:

Table C below shows good stability (survival for greater than 56 days) in elevated (30°C) temperature storage using the method of the invention.

**Table C**

| | **Time (days)** | **Strips inoculated with single colonies** (cfu/strip) | **Strips inoculated with stationery culture** (cfu/strip) | **Strips inoculated with concentrated cells** (cfu/strip) | **Control D No Inoculation** (cfu/strip) |
|---|---|---|---|---|---|
| **Inoculum added to strip** | 0 | **1.2E+07** | **7.0E+04** | **3.4E+06** | No colonies observed. |
| | | | | | |
| **Cells recovered from storage strip** | 0 (1 hr after inoculation) | 7.0E+06 | 3.8E+03 | 4.6E+05 | No further tests carried out |
| | 8 | 9.OE+05 | 3.9E+02 | 4.7E+04 | |
| | 14 | 1.0E+06 | 7.4E+02 | 4.3E+04 | |
| | 22 | 1.7E+06 | 5.1E+02 | 4.7E+04 | |
| | 56 | 1.1 E+06 | 2.0E+03 | 2.4E+05 | |

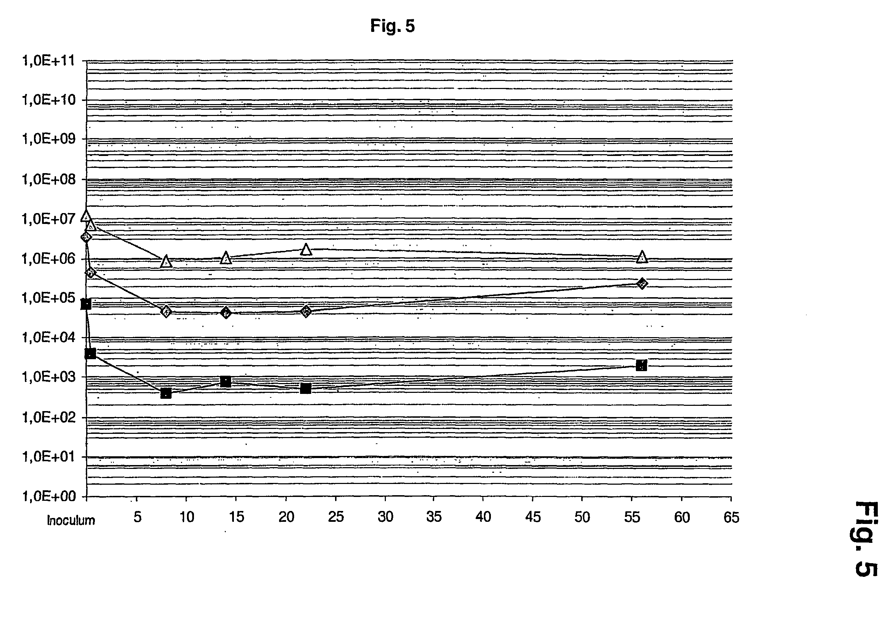

A graphical view of these results can be found in Figure 5. On the X-axis the time is given in days. On the Y-axis, the cfu per strip is shown. The triangles quadrats, and diamonds show the data obtained with the Single colony, Culture and Concentrated cell inoculums respectively.

### Experiment 4 To show cell survival with Pseudomonas aeruginosa ATCC 27853 on the test strip, using 3 inoculum types; Colony, Culture and Concentrated cells.

### Preparation of the strip devices

As detailed in Experiment 1 with the following exception;

The self adhesive desiccant (CSP Technologies, ACTIV-STRIP, M-0002-58), was removed from the protective foil pouch, and cut into pieces of 44.0mm x ~ 12.6mm x 0.30mm.

### Preparation of the inocula

*Pseudomonas aeruginosa* ATCC-27853 was obtained as TWISTER 0026 (PROLAB Diagnostics) from which a seed stock was prepared. The TWISTER was rehydrated using the rehydration solution provided and streaked onto Nutrient Agar (Merck KGaA, Art. no.1.05450) at 37°C for 16 hours.

A well isolated colony was selected from the agar plate, transferred to a vial containing cryobeads (Microbank, PROLAB Diagnostics, Art. No. PL160) and emulsified in the protective medium. The vial was inverted several times and as much of the protectant medium as possible was removed by aspiration. The vial was transferred to -70°C immediately after inoculation.

The organism was streaked from this cryobead stock onto a Nutrient Agar plate using conventional methods to ensure growth of individual separate single colonies of bacteria, and incubated at 37°C for 16 hours. This agar plate was used to provide single colonies for the inoculation of the strips (the Colony inoculum), and to inoculate broth to be used for the Culture and Concentrated cell inoculums.

A single colony from the plate prepared above was used to inoculate 50ml Nutrient Broth (Nutrient Broth No.2 Merck KGaA, Art. no. 11013610g/l) in a 100ml Sterilin pot (Sterilin Art. No. 185AM), and incubated at 37°C for 16 hours. The optical density, OD₆₀₀ₙₘ was measured (Pharmacia LKB, Novaspec II Visible Spectrophotometer) to be 0.48. The culture was placed on ice for 15 minutes. An aliquot of approximately 4ml of this culture was maintained on ice and used to inoculate the strips (the Culture inoculum).

An aliquot of 45ml of the chilled culture was transferred to a pre-chilled centrifuge tube (Falcon, Art. no. 352070). The cells were collected by centrifugation in an 5804R Eppendorf centrifuge using rotor type F34-6-38 and inserts (Eppendorf, Art No 5804 774000), at 6000rpm, 4°C for 5 minutes. An aliquot of approximately 5ml of the supernatant (ie spent media) was decanted into a Universal vial and the remainder discarded, taking care not to disturb the pellet. The Universal containing the supernatant was placed on ice. The pellet was resuspended in 0.45ml of the supernatant by gentle aspiration using a 1 ml sterile plastic pasteur pipette. The resuspended pellet was pooled, and returned to the ice bath. This concentrated cell suspension was used to inoculate the strips (the Concentrated cell inoculum).

The viability of the cells in the Culture inoculum and the Concentrated cell inoculum were determined at this point. This was carried out as described in Experiment 1

### Inoculation of the strips

### Inoculation using colonies

As described in Experiment 2 with the following exceptions:
Nutrient Broth was used as the diluent, and Nutrient Agar as the growth medium. The plates were incubated at 37°C for at least 16 hours.

### Inoculation using the Culture and the Concentrated cell suspension

As described for Experiment 1.

This procedure was carried out for each of the inoculum types.

### To determine the viable cell number on the strip.

As described for Experiment 1.

### The plates were incubated at 37°C for at least 16 hours.

### Control E - Strips prepared as described above without inoculation

As described for Experiment 2.

### Results:

Table D below shows good stability (survival for greater than 56 days) in elevated (30°C) temperature storage using the method of the invention.

**Table D**

| | **Time (days)** | **Strips inoculated with single colonies** (cfu/strip) | **Strips inoculated with stationery culture** (cfu/strip) | **Strips inoculated with concentrated cells** (cfu/strip) | **Control E No inoculation** (cfu/strip) |
|---|---|---|---|---|---|
| **Inoculum added to strip** | 0 | **4.1 E+07** | **3.8E+06** | **1.4E+08** | No colonies observed. |
| | | | | | |
| **Cells recovered from storage strip** | 0 (1 hr after inoculation) | 8.4E+06 | 1.7E+05 | 1.3E+07 | No further tests carried out |
| | 6 | | 2.4E+03 | 6.6E+05 | |
| | 7 | 1.0E+06 | | | |
| | 14 | 5.0E+05 | 2.3E+03 | 1.8E+06 | |
| | 21 | 6.7E+05 | 1.4E+03 | 1.6E+06 | |
| | 56 | 3.6E+04 | 5.3E+02 | 1.8E+05 | |

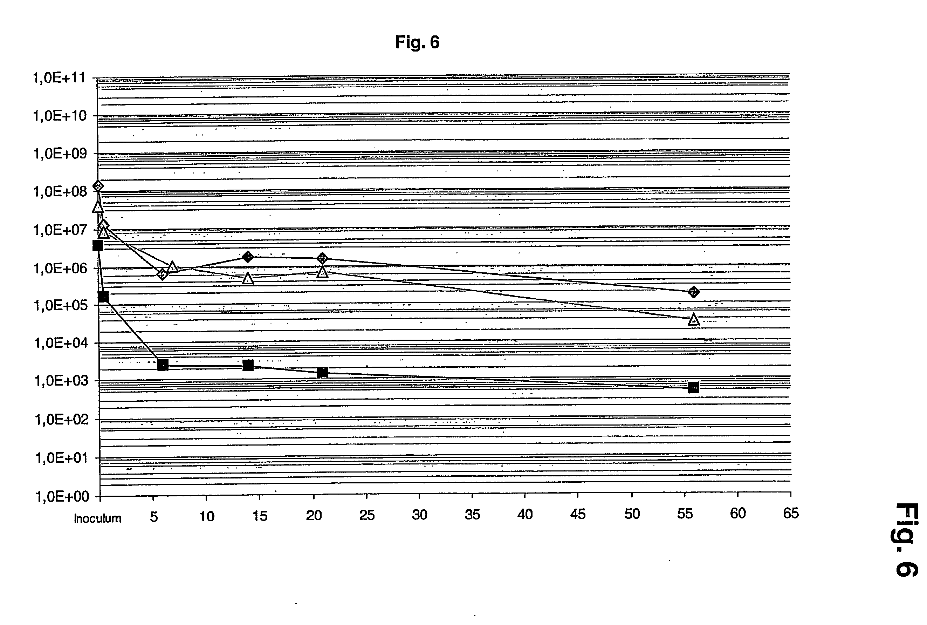

A graphical view of these results can be found in Figure 6. On the X-axis the time is given in days. On the Y-axis, the cfu per strip is shown. The triangles quadrats, and diamonds show the data obtained with the Singles colony, Culture and Concentrated cell inoculums respectively.

### Experiment 5 To show cell survival with Lactobacillus acidophilus ATCC 4356 on the test strip, using 3 inoculum types; Colony, Culture and Concentrated cells.

### Preparation of the strip devices

As described in Experiment 1.

### Preparation of the inocula

*Lactobacilus acidophilus* ATCC-4356 was obtained as TWISTER 0018 (PROLAB Diagnostics). The TWISTER was rehydrated using the rehydration solution provided and streaked onto MRS Agar (52.4g/l MRS Broth, Merck KGaA, Art. no. 11066, 14g/l Agar, Merck KGaA, Art. No. 101614). The agar plate was incubated at 35°C for 72 hours in a CO₂ enriched atmosphere.
This agar plate was used to provide single colonies for the inoculation of the strips (the Colony inoculum).

An aliquot of 100*µ*l of the rehydrated cells from the TWISTER was transferred to 50ml MRS Broth (52.4g/l MRS Broth, Merck KGaA, Art. no. 110661) in a 100ml Sterilin pot (Sterilin Art. No. 185AM), and incubated at 35°C for 72 hours. The optical density, OD₆₀₀ₙₘ was measured (Pharmacia LKB, Novaspec II Visible Spectrophotometer) to be 0.97. The culture was placed on ice for 15 minutes. An aliquot of approximately 4ml of this culture was maintained on ice and used to inoculate the strips (the Culture inoculum).

An aliquot of 45ml of the chilled culture was transferred to a pre-chilled centrifuge tube (Falcon, Art. no. 352070). The cells were collected by centrifugation in an 5804R Eppendorf centrifuge using rotor type F34-6-38 and inserts (Eppendorf, Art No 5804 774000), at 6000rpm, 4°C for 5 minutes. An aliquot of approximately 5ml of the supernatant (ie spent media) was decanted into a Universal vial and the remainder discarded, taking care not to disturb the pellet. The Universal containing the supernatant was placed on ice. The pellet was resuspended in 0.45ml of the supernatant by gentle aspiration using a 1ml sterile plastic pasteur pipette. The resuspended pellet was pooled, and returned to the ice bath. This concentrated cell suspension was used to inoculate the strips (the Concentrated cell inoculum).

The viability of the cells in the Culture inoculum and the Concentrated cell inoculum were determined at this point. Standard dilution series were prepared in MRS Broth and plated in duplicate onto MRS Agar. The plates were incubated at 35°C for at least 72 hours in a CO₂ enriched atmosphere, and the colonies counted.

### Inoculation of the strips

### Inoculation using colonies

As described for Experiment 2 with the following exceptions:
MRS Broth was used as the diluent, and MRS Agar as the growth medium. The plates were incubated at 35°C for at least 72 hours in a CO₂ enriched atmosphere.

### Inoculation using the culture and the concentrated cell suspension

As described for Experiment 1.

This procedure was carried out for each of the inoculum types.

### To determine the viable cell number on the strip.

As described for Experiment 1 with the following exceptions;

MRS broth was used as the diluent, and MRS Agar as the growth medium. The plates were incubated at 35°C for at least 72 hours in a CO₂ enriched atmosphere.

### Control F - Strips prepared as described above without inoculation

As described for Experiment 2.

### Results:

Table E below shows good stability (survival for greater than 56 days) in elevated (30°C) temperature storage.

**Table E**

| | **Time (days)** | **Strips inoculated with single colonies** (cfu/strip) | **Strips inoculated with stationery culture** (cfu/strip) | **Strips inoculated with concentrated cells** (cfu/strip) | **Control F No inoculation** (cfu/strip) |
|---|---|---|---|---|---|
| **Inoculum added to strip** | 0 | **9.6E+06** | **4.7E+05** | **2.2E+07** | No colonies observed. |
| | | | | | |
| **Cells recovered from storage strip** | 0 (1 hr after inoculation) | 3.6E+05 | 3.3E+04 | 1.6E+06 | No further tests carried out |
| | 7 | 1.6E+06 | 2.4E+04 | 8.2E+05 | |
| | 15 | 2.2E+05 | 5.7E+03 | 3.8E+05 | |
| | 21 | 3.1E+05 | 1.8E+04 | 1.6E+06 | |
| | 56 | 6.4E+05 | 8.8E+03 | 5.2E+05 | |

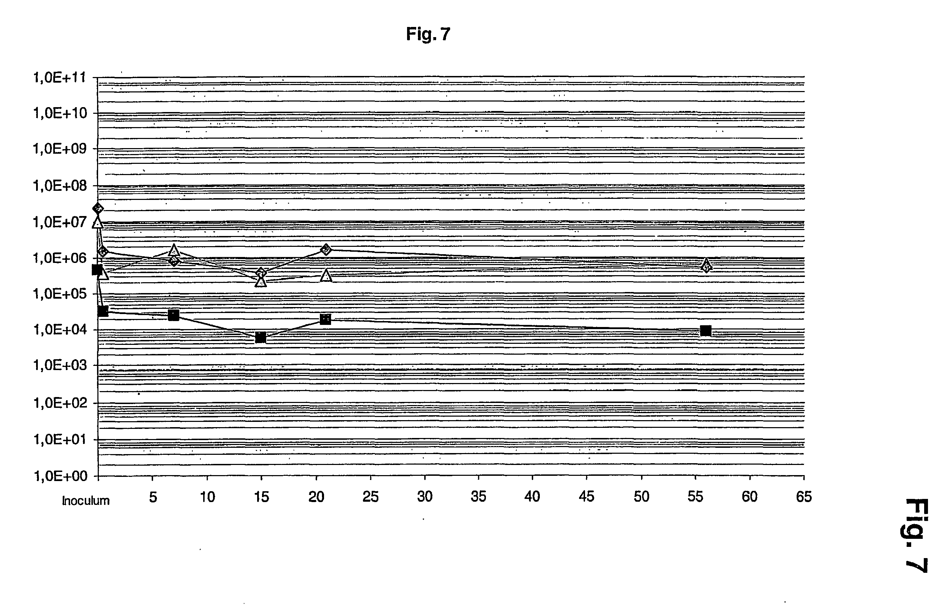

A graphical view of these results can be found in Figure 7. On the X-axis the time is given in days. On the Y-axis, the cfu per strip is shown. The triangles quadrats, and diamonds show the data obtained with the Singles colony, Culture and Concentrated cell inoculums respectively.

### Experiment 6 To show cell survival on the test strip with desiccant present in the system.

### Preparation of the strip devices

As described in Experiment 1 with the following exceptions:
The desiccant pieces were without adhesive. The foil pouch was sealed using a soldering iron (Weller WHS40) at a temperature of 350°C

### Preparation of the inoculum

*E.coli* ATCC-25922 was obtained as TWISTER 0014 (PROLAB Diagnostics) from which a seed stock was prepared. The TWISTER was rehydrated using the rehydration solution provided and streaked onto Nutrient Agar (Merck KGaA, Art. no.1.05450) at 37°C for 16 hours.

A well isolated colony was selected from the agar plate, transferred to a vial containing cryobeads (Microbank, PROLAB Diagnostics, Art. No. PL160) and emulsified in the protective medium. The vial was inverted several times and as much of the protectant medium as possible was removed by aspiration. The vial was transferred to -70°C immediately after inoculation.

The organism was streaked from this cryobead stock onto a Nutrient Agar plate using conventional methods to ensure growth of individual separate single colonies of bacteria, and incubated at 37°C for 16 hours.

A single colony from the plate prepared above was used to inoculate 50ml Nutrient Broth (Nutrient Broth No.2 Merck KGaA, Art. no. 11013610g/l) in a 100ml Sterilin pot (Sterilin Art. No. 185AM), and incubated at 37°C for 16 hours. The optical density, OD₆₀₀ₙₘ was measured (Pharmacia LKB, Novaspec II Visible Spectrophotometer) to be 0.52. The culture was placed on ice for 15 minutes. This culture was used to inoculate the strips.

The viability of the cells in the culture was determined at this point. This was carried out as described in Experiment 1

### Inoculation of the strips

### Inoculation using the culture

As described for Experiment 1with the following exception;
Immediately after inoculation, the primary seal was replaced to seal the access window, and a secondary seal of Aluminium foil tape, 50 micron, (RS Art. No 408-9574) was placed over this primary seal.

### To determine the viable cell number on the strip.

### As described for Experiment 1

### Control G - Strips prepared as described above without desiccant

Strip devices were prepared and tested as described in the above method, except for the omission of the desiccant.

### Results:

Table F The surprising result can be seen from Table F below that good stability (survival for greater than 14 days (in elevated (30°C) temperature storage) was only achieved with the incorporation of desiccant within the device. The bacteria inoculated into the device did not survive for greater than seven days in the absence of desiccant in the device.

**Table F**

| | **Time (days)** | **Strips prepared with desiccant** (cfu/strip) | **Control G - Strips prepared without desiccant** (cfu/strip) |
|---|---|---|---|
| **Inoculum added to strip** | **0** | 2.1 E+06 | 2.1 E+06 |
| | | | |
| **Cells recovered from storage strip** | 0 (1 hr after inoculation) | 2.4E+04 | 2.2E+04 |
| | 7 | 4.0E+03 | No colonies detected |
| | 14 | 7.5E+03 | No colonies detected |
| | 21 | 1.0E+04 | No further tests carried out |

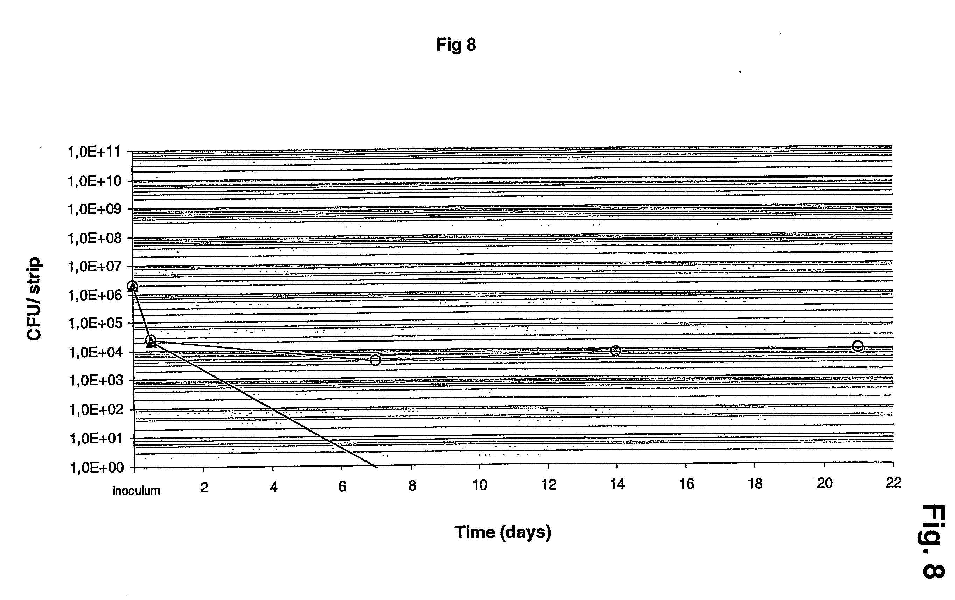

A graphical view of these results can be found in Figure 8. On the X-axis the time is given in days. On the Y-axis, the cfu per strip is shown. The circles show the data obtained for the strips prepared with desiccant, and the triangles for those without desiccant.

## Claims

1. Re-sealable strip device suitable for drying and storage of viable micro-organisms without losing viability comprising
- a strip comprising a water absorbent preparation containing an amount of at least one water absorbent substance, which is able to absorb the water contained in the chosen volume of added micro- organism preparation such that the final amount of water after addition of the cells in the preparation(s) is less than 10 % w/w and at least one protective substance
- a re-sealable housing which when being sealed provides a closed, water-impermeable system around the strip.

2. Re-sealable strip device according to claim 1, **characterised in that** the water absorbent preparation incorporates one or more substances that have both protective and water absorbent properties.

3. Re-sealable strip device according to claim 1 or 2, **characterised in that** the primary water absorbent preparation further comprises one or several absorbent foam or fibre-based pads or other matrices.

4. Re-sealable strip device according to one or more of claims 1 to 3, **characterised in that** the water absorbent preparation is in contact with or in proximity to a secondary water absorbent preparation.

5. Re-sealable strip device according to one or more of claims 1 to 4, **characterised in that** the secondary water absorbent preparation has the format of one or several absorbent beads, tablets, plugs, foam or fibre-based pads enclosed within the strip device.

6. Re-sealable strip device according to one or more of claims 1 to 5, **characterised in that** the re-sealable housing is a foil pouch.

7. Re-sealable strip device according to one or more of claims 1 to 6, **characterised in that** the re-sealable housing comprises one or more access windows over each of which there is a water impermeable cover that can be opened and re-sealed such that once re-sealed the device regains its water impermeable properties.

8. Re-sealable strip device according to one or more of claims 1 to 7, **characterised in that** the re-sealable housing has a label.

9. Re-sealable strip device according to one or more of claims 1 to 8, **characterised in that** the re-sealable housing has a secondary tear or peel seal which can be opened in order to aseptically remove the strip.

10. Process for the drying and storage of viable micro-organisms without losing viability, by
a) providing a re-sealable strip device according to one or more of claims 1 to 9;
b) opening a re-sealable access window cover in the housing of the strip device to gain access to the strip;
c) adding the micro-organism preparation to be dried through the said window on to the water absorbent preparation located on the strip;
d) sealing the strip device by closing the re-sealable window cover on said housing;
e) storing the strip device,
whereby the amount of the micro-organism preparation and the amount of the water absorbent preparation within the device are chosen such that after the addition of the micro-organism preparation the water absorbent preparation is able to absorb the water contained in the micro-organism preparation and remain substantially dry, which means that the final amount of water after addition of the cells in the preparation(s) is less than 10 % w/w.

11. Process according to claim 10, **characterised in that**, after step e), the micro-organisms are recovered by
a) opening the strip device and removing the strip from the housing;
b) rehydrating the micro-organisms.

12. Process according to claim 10 or 11, **characterised in that**, in step e), the strip device is stored at a temperature between 0°C and 30°C.

13. A kit for drying and storage of micro-organisms at least comprising a re-sealable strip device according to one or more of claims 1 to 9.

## Patentansprüche

1. Wiederverschließbare Streifenvorrichtung, die sich zum Trocknen und Lagern von lebensfähigen Mikroorganismen ohne Verlust der Lebensfähigkeit eignet, enthaltend
- einen Streifen enthaltend eine wasserabsorbierende Zubereitung, welche eine Menge mindestens einer wasserabsorbierenden Substanz enthält, die in der Lage ist, das in dem gewählten Volumen an zugesetzter Mikroorganismenzubereitung enthaltene Wasser so zu absorbieren, dass die Endmenge an Wasser in der/den Zubereitung(en) nach Zugabe der Zellen weniger als 10% w/w beträgt, und mindestens eine Schutzsubstanz
- einen wiederverschließbaren Behälter, der im verschlossenen Zustand ein geschlossenes, wasserundurchlässiges System um den Streifen bereitstellt.

2. Wiederverschließbare Streifenvorrichtung nach Anspruch 1, **dadurch gekennzeichnet, dass** die wasserabsorbierende Zubereitung eine oder mehrere Substanzen beinhaltet, die sowohl schützende als auch wasserabsorbierende Eigenschaften besitzen.

3. Wiederverschließbare Streifenvorrichtung nach Anspruch 1 oder 2, **dadurch gekennzeichnet, dass** die primäre wasserabsorbierende Zubereitung weiterhin ein oder mehrere absorbierende Schaumstoff- oder faserbasierende Polster oder andere Matrizes enthält.

4. Wiederverschließbare Streifenvorrichtung nach einem oder mehreren der Ansprüche 1 bis 3, **dadurch gekennzeichnet, dass** sich die wasserabsorbierende Zubereitung im Kontakt mit oder in der Nähe von einer sekundären wasserabsorbierenden Zubereitung befindet.

5. Wiederverschließbare Streifenvorrichtung nach einem oder mehreren der Ansprüche 1 bis 4, **dadurch gekennzeichnet, dass** die sekundäre wasserabsorbierende Zubereitung in Form von einer oder mehreren in der Streifenvorrichtung eingeschlossenen absorbierenden Perlen, Tabletten, Pfropfen, Schaumstoff- oder faserbasierenden Polstern vorliegt.

6. Wiederverschließbare Streifenvorrichtung nach einem oder mehreren der Ansprüche 1 bis 5, **dadurch gekennzeichnet, dass** der wiederverschließbare Behälter ein Folienbeutel ist.

7. Wiederverschließbare Streifenvorrichtung nach einem oder mehreren der Ansprüche 1 bis 6, **dadurch gekennzeichnet, dass** der wiederverschließbare Behälter ein oder mehrere Zugangsfenster enthält, über denen sich jeweils eine wasserundurchlässige Abdeckung befindet, die so geöffnet und wieder verschlossen werden kann, dass die Vorrichtung nach dem Wiederverschließen ihre wasserundurchlässigen Eigenschaften zurückerhält.

8. Wiederverschließbare Streifenvorrichtung nach einem oder mehreren der Ansprüche 1 bis 7, **dadurch gekennzeichnet, dass** der wiederverschließbare Behälter ein Etikett aufweist.

9. Wiederverschließbare Streifenvorrichtung nach einem oder mehreren der Ansprüche 1 bis 8, **dadurch gekennzeichnet, dass** der wiederverschließbare Behälter einen sekundären aufreißbaren oder abziehbaren Verschluss aufweist, der geöffnet werden kann, um den Streifen aseptisch zu entnehmen.

10. Verfahren zum Trocknen und Lagern von lebensfähigen Mikroorganismen ohne Verlust der Lebensfähigkeit, durch
a) Bereitstellen einer wiederverschließbaren Streifenvorrichtung nach einem oder mehreren der Ansprüche 1 bis 9;
b) Öffnen einer wiederverschließbaren Zugangsfensterabdeckung im Behälter der Streifenvorrichtung, um Zugang zum Streifen zu erhalten;
c) Zugeben der zu trocknenden Mikroorganismenzubereitung durch das besagte Fenster auf die auf dem Streifen befindliche wasserabsorbierende Zubereitung;
d) Verschließen der Streifenvorrichtung durch Schließen der wiederverschließbaren Fensterabdeckung am genannten Behälter;
e) Lagern der Streifenvorrichtung;
wobei die Menge der Mikroorganismenzubereitung und die Menge der wasserabsorbierenden Zubereitung in der Vorrichtung so gewählt werden, dass die wasserabsorbierende Zubereitung nach der Zugabe der Mikroorganismenzubereitung in der Lage ist, das in der Mikroorganismenzubereitung enthaltene Wasser zu absorbieren und im Wesentlichen trocken zu bleiben, so dass die Endmenge an Wasser in der/den Zubereitung(en) nach Zugabe der Zellen weniger als 10% w/w beträgt.

11. Verfahren nach Anspruch 10, **dadurch gekennzeichnet, dass**, nach Schritt e), die Mikroorganismen wiedergewonnen werden durch
a) Öffnen der Streifenvorrichtung und Entfernen des Streifens aus dem Behälter;
b) Rehydratisieren der Mikroorganismen.

12. Verfahren nach Anspruch 10 oder 11, **dadurch gekennzeichnet, dass**, in Schritt e), die Streifenvorrichtung bei einer Temperatur zwischen 0°C und 30°C gelagert wird.

13. Kit zum Trocknen und Lagern von Mikroorganismen, mindestens enthaltend eine wiederverschließbare Streifenvorrichtung nach einem oder mehreren der Ansprüche 1 bis 9.

## Revendications

1. Dispositif de bande rescellable convenant pour le séchage et le stockage de micro-organismes viables sans perte de viabilité, comprenant :
- une bande comprenant une préparation absorbeuse d'eau contenant une certaine quantité d'au moins une substance absorbeuse d'eau, qui peut absorber l'eau contenue dans le volume choisi d'une préparation de micro-organismes ajoutée de telle sorte que la quantité finale d'eau après addition des cellules dan la ou les préparation(s) soit inférieure à 10% en poids/poids et d'au moins une substance de protection ; et
- un conteneur rescellable qui, lorsqu'il est scellé, constitue un système imperméable à l'eau et fermé, autor de la bande.

2. Dispositif de bande rescellable selon la revendication 1, **caractérisé en ce que** la préparation absorbeuse d'eau incorpore une ou plusieurs substances qui présentent à la fois de propriétés de protection et d'absorption d'eau.

3. Dispositif de bande rescellable selon la revendication 1 ou 2, **caractérisé en ce que** la préparation absorbeuse d'eau primaire comprend en outre un ou plusieurs patins absorbeurs en mousse ou à base de fibres ou d'autres matrices.

4. Dispositif de bande rescellable selon une ou plusieurs des revendications 1 à 3, **caractérisé en ce que** la préparation absorbeuse d'eau est en contact avec une préparation absorbeuse d'eau secondaire ou est à proximité de celle-ci.

5. Dispositif de bande rescellable selon une ou plusieurs des revendications 1 à 4, **caractérisé en ce que** la préparation absorbeuse d'eau secondaire présente le format constitué par un ou plusieurs éléments pris parmi des perles, des tablettes, des bouchons, des patins en mousse ou à base de fibres qui sont absorbants, renfermés à l'intérieur du dispositif de bande.

6. Dispositif de bande rescellable selon une ou plusieurs des revendications 1 à 5, **caractérisé en ce que** le conteneur rescellable est une poche souple.

7. Dispositif de bande rescellable selon une ou plusierus des revendications 1 à 6, **caractérisé en ce que** le conteneur rescellable comprend une ou plusieurs fenêtres d'accès sur chacune desquelles il y a un couvercle imperméable à l'eau qui peut être ouvert et rescellé de telle sorte qu'une fois qu'il est rescellé, le dispositif retrouve ses propriétés d'imperméabilité à l'eau.

8. Dispositif de bande rescellable selon une ou plusieurs des revendications 1 à 7, **caractérisé en ce que** le conteneur rescellable comporte une étiquette.

9. Dispositif de bande rescellable selon une ou plusieurs des revendications 1 à 8, **caractérisé en ce que** le conteneur rescellable comporte une scellement secondaire déchirable ou pelable qui peut être ouvert afin d'enlever de façon aseptique la bande.

10. Procédé pour le séchage et le stockage de micro-organismes viables sans perte de viabilité, en
a) fournissant un dispositif de bande rescellable selon une ou plusieurs des revendications 1 à 9 ;
b) ouvrant un couvercle de fenêtre d'accès rescellable dans le conteneur du dispositif de bande afin d'obtenir un accès à la bande ;
c) ajoutant la préparation de micro-organismes à sécher au travers de ladite fenêtre sur la préparation absorbeuse d'eau localisée sur la bande ;
d) scellant le dispositif de bande en fermant le couvercle de fenêtre rescellable sur ledit conteneur ;
e) stockant le dispositif de bande;
et ainsi, la quantité de la préparation de micro-organismes et la quantité de la préparation absorbeuse d'eau à l'intérieur du dispositif sont choisies de telle sorte qu'après l'ajout de la préparation de micro-organismes, la préparation absorbeuse d'eau puisse absorber l'eau contenue dans la préparation de micro-organismes et puisse rester sensiblement sèche, ce qui signifie que la quantité finale d'eau après ajout des cellules dans la ou les préparation(s) est inférieure à 10% en poids/poids.

11. Procédé selon la revendication 10, **caractérisé en ce que**, après l'étape e), les micro-organismes sont recouverts en
a) ouvrant le dispositif de bande et enlevant la bande du conteneur ; et
b) réhydratant les micro-organismes.

12. Procédé selon la revendication 10 ou 11, **caractérisé en ce que** au niveau de l'étape e), le dispositif de bande est stocké à une température entre 0°C et 30°C.

13. Kit pour le séchage et le stockage de micro-organismes, comprenant au moins un dispositif de bande rescellable selon une ou plusieurs des revendications 1 à 9.
